# EUROPÄISCHE PATENTSCHRIFT

(11) **EP 0 729 736 B1**
(45) Veröffentlichungstag und Bekanntmachung des Hinweises auf die Patenterteilung: **09.05.2001**
(21) Anmeldenummer: 96101894.2
(22) Anmeldetag: 09.02.1996
(51) Int. Cl.: A61G 13/10, A61B 19/08

(54) **Operationstischbezug sowie Verfahren zu seiner Herstellung**
Cover for operating table and method of manufacture thereof
Couverture pour table d'opération et procédé pour sa fabrication

(30) Priorität: 22.02.1995 DE 19506046
(43) Veröffentlichungstag der Anmeldung: 04.09.1996
(73) Patentinhaber: GFM Maschinenbau GmbH, 45739 Oer-Erkenschwick (DE)
(72) Erfinder: Gawarecki, Herbert, D-45721 Haltern (DE)
(74) Vertreter: Eichelbaum, Lambert, Dipl.-Ing.

(56) Entgegenhaltungen:
- DE-A- 3 519 705
- DE-A- 3 715 691
- DE-C- 4 033 243

## Beschreibung

Die Erfindung betrifft einen Operationstischbezug, bestehend aus einem Folienschlauch von thermoplastischem Kunststoff mit einem geschlossenen Bodenbereich und einem offenen, mit einem umlaufenden Stulpenrand versehenen Kopfbereich, dessen Seitenkanten durch Schweißnähte verschlossen sind sowie ein Verfahren zur Herstellung eines solchen Operationstischbezuges.

Bei einem bekannten Operationstischbezug dieser Art gemäß der EP 0 290 738 B1 und der DE 37 15 691 A1 ist die Breite des Stulpenrandes am Kopfende gegenüber den übrigen Bereichen des Schlauches größer gestaltet und gegenüber den Schweißnähten der Seitenkanten nach außen versetzt. Durch diese Gestaltung ist dieser Operationstischbezug bei seiner Herstellung mit dem Nachteil relativ langer Abfallstreifen behaftet, die sich von der Unterkante des Stulpenrandes bis zum Bodenbereich erstrecken. Dabei ist jeder Hersteller eines solchen Operationstischbezuges bestrebt, ihn einerseits möglichst enganliegend und trotzdem bequem über eine als Operationstisch dienende Auflagefläche stülpen zu können und ihn andererseits mit möglichst geringen Abfallabschnitten zu behaften. Diese beiden Forderungen stehen in einem gewissen Gegensatz zueinander. Aus diesem Bestreben heraus erklärt sich, daß ein Operationstischbezug der vorgenannten Druckschriften den weiteren Handhabungsnachteil aufweist, daß sich zwar sein Stulpenrand relativ leicht über eine als Operationstisch dienende Auflagefläche stülpen läßt, hingegen die nachfolgende lange Strecke unterhalb seines Stulpenrandes bis zum Bodenbereich unter Überwindung relativ großer Friktionskräfte über die Auflagefläche gezogen werden muß. Diese Friktion wird noch durch die unangenehme Eigenschaft der Kunststoffolie verstärkt, die sich bei diesem Überstülpvorgang häufig in Teilbereichen ihrer Innenseiten an der Auflagefläche festsaugt.

Ein weiterer Operationstischbezug anderer Art ist aus der DE 35 19 705 A1 bekannt, bei welchem die größere Breite des Stulpenrandes dadurch erhalten wird, daß die Innenweite des Folienschlauches im Bereich des Stulpenrandes gegenüber den übrigen Bereichen des Folienschlauches bleibend aufgeweitet wird. Diese Aufweitung des Stulpenrandes erfordert eine maschinelle Einrichtung innerhalb des Folienschlauches, die schwierig zu handhaben ist und mit noch größerer Schwierigkeit eine stets gleichbleibende Aufweitung erreichen läßt, weil die Elastizitätsgrenze des Folienmaterials zumindest in Teilbereichen überschritten werden muß. Weitere Abhängigkeiten ergeben sich aus dem Werkstoff des Folienmaterials bei der jeweiligen Fertigungstemperatur.

Und schließlich ist aus der DE 40 33 243 C1 ein Operationstischbezug anderer Gattung bekannt geworden, dessen Stulpenrand die gleiche Breite wie der Folienschlauch aufweist und der mit seinem freien Ende zu einem mindestens zwei Lagen umfassenden Stulpenrand mit der außenliegenden Lage in Richtung auf die Öffnung im Kopfbereich umgefaltet ist. Dieser Operationstischbezug läßt sich zwar rascher und mit geringerer Kraftanstrengung über den Operationstisch streifen als alle vorbeschriebenen, konnte sich jedoch in der Praxis nicht durchsetzen, weil das Krankenhauspersonal in aller Regel nicht bereit ist, einmal eingeübte Handgriffe eines bekannten Operationstischbezuges im Hinblick auf ein anderes Produkt zu ändern.

Von diesem Stand der Technik ausgehend, liegt der Erfindung die Aufgabe zugrunde, einen Operationstischbezug der eingangs genannten Gattung zu schaffen, der sich trotz seiner abfallärmeren Fertigung durch eine einfachere Handhabung beim Überstreifen über die Auflagefläche eines Operationstisches auszeichnet.

Diese Aufgabe wird in Verbindung mit dem eingangs genannten Gattungsbegriff erfindungsgemäß dadurch gelöst, daß die Seitenkanten in der Nähe des Bodenbereiches und unmittelbar unterhalb des Stulpenrandes mit nach innen gerichteten Versatzbereichen versehen sind und der Stulpenrand die gleiche Breite wie der zwischen den beiden Versatzbereichen befindliche Mittenbereich des Folienschlauches aufweist. Durch diese Ausbildung weist der Folienschlauch nunmehr nicht mehr in seinem Mittenbereich, sondern lediglich in seinen Versatzbereichen Engstellen auf, wohingegen der gesamte, relativ lange Mittenbereich ebenso wie der Stulpenrand sich bequem über die Auflagefläche eines Operationstisches ziehen lassen. Ferner zeichnet sich dieser Operationstischbezug durch eine erheblich abfallärmere Fertigung aus, da sich die Abfallstücke auf eine Größe beschränken, die der Größe der relativ kleinen Versatzbereiche entsprechen.

In vorteilhafter Weiterbildung der Erfindung ist die Länge des Versatzbereiches in den Seitenkanten unterhalb des Stulpenrandes geringfügig größer als die Länge des Stulpenrandes, wohingegen die Länge der Versatzbereiche der Seitenkanten in der Nähe des Bodenbereiches entweder gleich oder geringfügig kleiner oder größer als die Länge des Stulpenrandes ist. Infolge dieser Abstimmung der Längen der Versatzbereiche und des Stulpenrandes kann der Bodenbereich relativ leicht unter den Stulpenrand geschoben werden, bevor er durch weitere Quer- und Längsfalzungen zu einem anwendungsgerechten Faltpäckchen verkleinert wird. Aus dem gleichen Grunde ist die Breite des Versatzbereiches unterhalb des Stulpenrandes gleich der Breite des Versatzbereiches in der Nähe des Bodenbereiches gestaltet.

Zur Verringerung einer Einreißgefahr unter Kerbwirkungen gehen die Enden der Versatzbereiche zu den angrenzenden, vorspringenden Seitenkanten des Mittenbereiches und des Stulpenrandes in Form eines Viertelkreises über. Je nach Herstellungsart ist der Bodenbereich des Operationstischbezuges entweder durch eine Faltkante oder durch eine Schweißnaht verschlossen.

Die Operationstischbezüge gemäß der EP 0 290 738 B1 und der DE 37 15 691 A1 werden aus einem Folienschlauch aus thermoplastischem Kunststoff hergestellt, der an der den Bodenbereich des Operationstischbezuges bildenden Längskante geschlossen und an der den Kopfbereich bildenden Längskante geöffnet ist und der in Transportlängsrichtung durch Vorzugsrollen oder Bänder gezogen, in regelmäßigen Abständen auf einer Außenseite mit Faservlies-Zuschnitten und sodann mit quer zur Transportrichtung verlaufenden Schweißnähten und Ausnehmungen versehen wird, wonach der Folienschlauch nach einer Längsfalzung im Mittenbereich sowie durch zwei zu entgegengesetzten Seiten umgeschlagenen Längsfalze an seinem Kopfbereich mit einem Stulpenrand versehen wird, dessen Seitenkanten durch je eine Schweißnaht verbunden werden. Bei diesem vorbekannten Verfahren werden nach dem Aufbringen der Faservlies-Zuschnitte, die bei einer steigenden Anzahl von Operationstischbezügen nicht mehr erforderlich sind, die quer zur Transportrichtung verlaufenden Schweißnähte in den Folienschlauch eingebracht, die bis zur Unterkante des Stulpenrandes reichen. Zwischen dieser Unterkante des Stulpenrandes und dem Bodenbereich weisen die benachbarten Zuschnitte für die Operationstischbezüge keine Verbindung mehr auf. Nach einer Längsfalzung im Mittenbereich sowie nach den beiden zu entgegengesetzten Seiten umgeschlagenen Längsfalzen an seinem Kopfbereich weist der Folienschlauch zwischen den einzelnen Zuschnitten für die Operationstischbezüge lediglich noch an seinem Kopfbereich eine Verbindung auf. Bevor die Seitenkanten des Stulpenrandes durch je eine Schweißnaht miteinander verbunden und zugleich die Zuschnitte dadurch voneinander getrennt werden, kann es durch Friktionen sowie Massenträgheiten während des Längstransportes zu einem Verzug eines nachfolgenden Zuschnittes kommen, wodurch eine Zusammenschweißung eines Kopfbereiches eines Operationstischbezuges mit einem Bodenbereich vorausgehenden Operationstischbezuges zu Ausschußprodukten führt.

Zur Vermeidung derartiger Ausschußprodukte sowie zur Herstellung der erfindungsgemäßen Operationstischbezüge ist das erfindungsgemäße Verfahren durch folgende Merkmale gekennzeichnet:
a) Der Folienschlauch wird zur Bildung von Versatzbereichen an den Seitenkanten des Operationstischbezuges unterhalb des Stulpenrandes und in der Nähe des Bodenbereiches einer Trennschweißung unterzogen,
b) im Mittenbereich der Seitenkanten zwischen den beiden Versatzbereichen wird der Folienschlauch mit einer quer zu seiner Längstransportrichtung verlaufenden Perforationsschweißnaht versehen und zu deren beiden Seiten durch Schweißen verschlossen, und
c) der Stulpenrand wird vor Trennung der einzelnen Operationstischbezüge an seinen beiden Seitenkanten mit Perforationsschweißnähten versehen und zu beiden Seiten davon durch Schweißnähte verschlossen.

Durch das Merkmal a) werden die relativ kleinflächigen Versatzbereiche mittels einer Trennschweißung hergestellt, wohingegen durch die Merkmale b) und c) die einzelnen Zuschnitte der Operationstischbezüge bis zu ihrer Trennung sowohl im relativ langen Mittenbereich als auch am Stulpenrand durch je eine Perforationsschweißnaht miteinander verbunden bleiben. Dadurch ist bis zu ihrer Trennung ein gleichmäßiger sowie verzugsfreier Transport der einzelnen noch zusammenhängenden Operationstischbezüge gewährleistet.

Die Perforationsschweißnähte im Mittenbereich und am Stulpenrand der Operationstischbezüge werden vorteilhaft gleichzeitg getrennt.

Nach einer ersten Ausführungsform werden die Operationstischbezüge nach ihrer Trennung durch weitere Längs- und Querfalzungen zu einem anwendungsgerechten Faltpäckchen verkleinert, so, wie sie bislang nach dem Herstellungsverfahren der Operationstischbezüge der EP 0 290 738 B1 und der DE 37 15 691 A1 bekannt und diese seit vielen Jahren in dieser Form auf dem Markt erhältlich sind.

Nach einer zweiten, besonders vorteilhaften Ausführungsform des erfindungsgemäßen Verfahrens können die zusammenhängenden Operationstischbezüge nach Herstellung der Perforationsschweißnähte im Mittenbereich und am Stulpenrand auf eine Rolle aufgewickelt werden, von welcher sie nacheinander an ihren Perforationsschweißnähten im Mittenbereich und am Stulpenrand abreißbar sind. Das Aufwickeln der Rolle erspart ein zeitaufwendiges Auspacken und Auseinanderfalten bei der bekanntn Setverpackung.

Das erfindungsgemäße Verfahren ist sowohl bezüglich des Zeitpunktes und Ortes der einzelnen Falzungen als auch bezüglich der zeitlichen Verfahrensanordnung der Perforationsschweißnähte in der Nähe des Stulpenrandes variabel. So ist es in Weiterbildung des Merkmales c) auch möglich, daß der Stulpenrand erst nach einer weiteren Längsfalzung unter Zwischenschiebung eines schützenden Metallbleches zwischen dem Mittenbereich und dem darauf längsgefalzten Stulpenrand an dessen Seitenkanten mit einer Perforationsschweißnaht versehen und beidseitig davon durch die Schweißnähte verschlossen wird. Bei diesem Verfahrensschritt schützt das Metallblech den Mittenbereich vor einer weiteren Schweißung während der Herstellung der Schweißnähte des Stulpenrandes.

Auch bei der Ausbildung des als Ausgangsmaterial dienenden Folienschlauches sind unterschiedliche Verfahren möglich. So wird der Folienschlauch nach einer ersten Alternative aus einer einlagigen Kunststoffolie hergestellt, die exakt in ihrer Mitte parallel zur Transportrichtung gefalzt wird. In diesem Fall bildet der Falzbereich den späteren Bodenbereich der einzelnen Operationstischbezüge.

Nach einer zweiten Alternative kann der Folienschlauch jedoch auch aus zwei kongruent übereinandergefügten Einzelfolien gebildet werden, die am Bodenbereich mit einer durchgehenden Schweißnaht parallel zur Transportrichtung versehen werden. Bei Anwendung dieses Verfahrens weisen die Operationstischbezüge an ihrem Bodenbereich gleichfalls eine Schweißnaht auf.

Mehrere Ausführungsbeispiele des neuen Operationstischbezuges, seiner Handhabung sowie sein Verfahren zur Herstellung ist in den Zeichnungen dargestellt. Dabei zeigen:
Fig. 1 die perspektivische Draufsicht auf einen Operationstischbezug in teilweise auseinandergezogener Lage,
Fig. 2 die perspektivische Ansicht von Fig. 1 nach dem teilweisen Überzug des Operationstischbezuges über eine Auflagefläche eines Operationstisches,
Fig. 3 die Seitenquerschnittsansicht des Operationstischbezuges von Fig. 2 vor seinem Überzug über die Auflagefläche,
Fig. 4 die perspektivische Querschnittsansicht von Fig. 3 nach teilweisem Überzug des Operationstischbezuges über die Auflagefläche,
Fig. 5 die perspektivische Draufsicht auf einen Operationstischbezug mit einem Versatzbereich unterhalb des Stulpenrandes und einem weiteren in der Nähe des Bodenbereiches, der durch eine Faltkante verschlossen ist,
Fig. 6 die Querschnittsansicht gemäß VI÷VI von Fig. 5,
Fig. 7 eine der Fig. 5 entsprechende Ansicht, jedoch mit einem durch eine Schweißnaht verschlossenen Bodenbereich,
Fig. 8 die Querschnittsansicht entsprechend den Pfeilen VIII÷VIII von Fig. 7,
Fig. 9 die perspektivische Draufsicht des Operationstischbezuges von Fig. 7 nach Herstellung des Stulpenrandes durch Umstülpen des Kopfbereiches,
Fig. 10 die Schnittansicht entlang der Linie X÷X von Fig. 9,
Fig. 11 die Draufsicht auf einen Folienschlauch zur Herstellung der Operationstischbezüge nach Einbringung der Versatzbereiche durch Trennschweißung und der ersten Längsfalzung,
Fig. 12 die Draufsicht von Fig. 11 mit weiteren Verfahrensschritten bis zur Ausbildung eines Operationstischbezuges in Form eines anwendungsgerechten Faltpäckchens,
Fig. 13 eine Schnittansicht durch die Schlauchfolie gemäß den Pfeilen XIII÷XIII von Fig. 11,
Fig. 14 eine Schnittansicht gemäß XIV÷XIV von Fig. 11,
Fig. 15 eine Schnittansicht gemäß den Pfeilen XV÷XV von Fig. 12,
Fig. 16 eine Schnittansicht gemäß den Pfeilen XVI÷XVI von Fig. 12,
Fig. 17 eine Schnittansicht gemäß XVII÷XVII von Fig. 12,
Fig. 18 die Ausschnittvergrößerung XVIII von Fig. 11 und zwei an ihren Perforationsschweißnähten im Mittenbereich und am Stulpenrand zusammenhängenden Operationstischbezügen,
Fig. 19 eine Schnittansicht entlang der Linie XIX÷XIX von Fig. 18 und
Fig. 20 eine der Fig. 11 entsprechende Ansicht eines Folienschlauches, der aus zwei kongruent übereinandergefügten Einzelfolien gebildet wird.

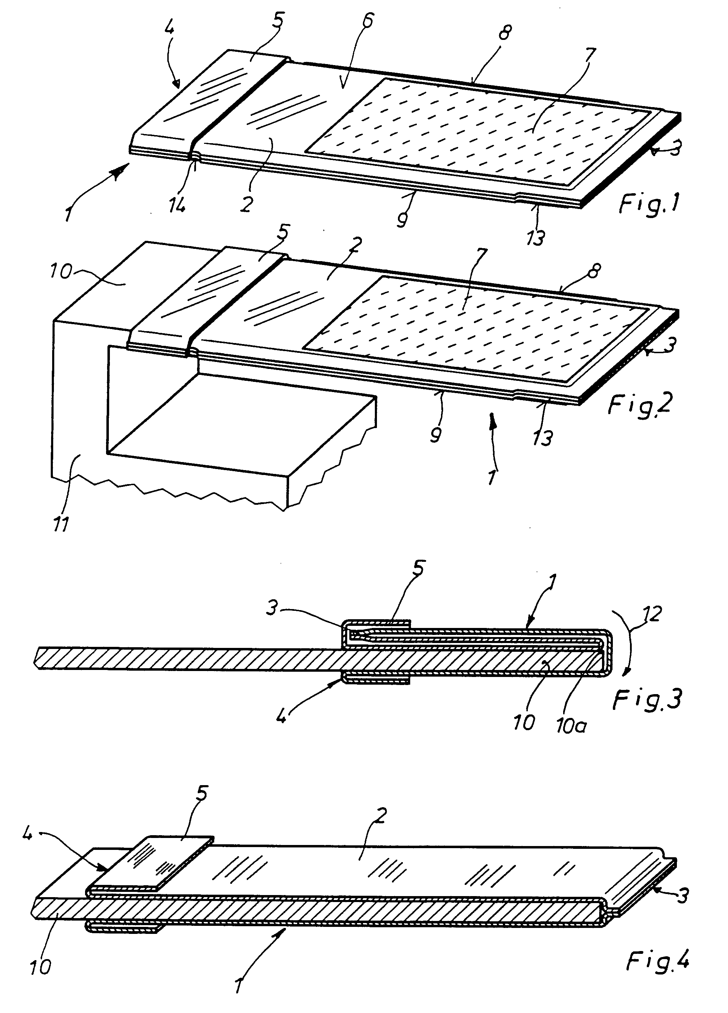

Gemäß den Figuren 1 bis 10 besteht der Operationstischbezug 1 aus einem Folienschlauch 2 von thermoplastischem Kunststoff mit einem geschlossenen Bodenbereich 3 und einem offenen, mit einem umlaufenden Stulpenrand 5 versehenen Kopfbereich 4. Auf einer Oberseite 6 ist der Operationstischbezug 1 mit einem saugfähigen Faservlies 7 belegt und an seinen Seitenkanten 8, 9 durch Schweißnähte verschlossen. Bei einer stetig ansteigenden Anzahl von Operationstischbezügen 1 sind die Faservliese 7 aufgrund neuartiger Operationstechniken mit speziellen Absaugvorrichtungen nicht mehr erforderlich.

Gemäß Fig. 2 ist der Operationstischbezug 1 über eine Auflagefläche 10 eines Operationstisches 11 gezogen.

Um die Oberfläche des Faservlieses 7 bis kurz vor ihrem bestimmungsgemäßen Gebrauch vor möglichen infektiösen Keimen zu schützen, wird der Operationstischbezug 1 gemäß Fig. 3 in der dort dargestellten, längsgefalteten Lage über die Auflagefläche 10 geschoben. Dabei greift die Krankenschwester beidseitig mit ihren Händen unter den Stulpenrand 5 unter Offenhaltung des Kopfbereiches 4 und schiebt den Operationstischbezug etwa bis zu seiner Mitte über die Auflagefläche 10. In dieser Lage ist das Faservlies 7 aufgrund seiner eingeklappten Lage vor Infektionskeimen geschützt. Beim weiteren Überschieben des Operationstischbezuges 1 über die Auflagefläche 10 rollt die weitere Hälfte des eingeklappt bleibenden Operationstischbezuges 1 in Richtung des Pfeiles 12 über die Kante 10a ab, bis gemäß Fig. 4 der Operationstischbezug 1 bis auf seinen Stulpenrand 5 über die Auflagefläche 10 gestreift ist.

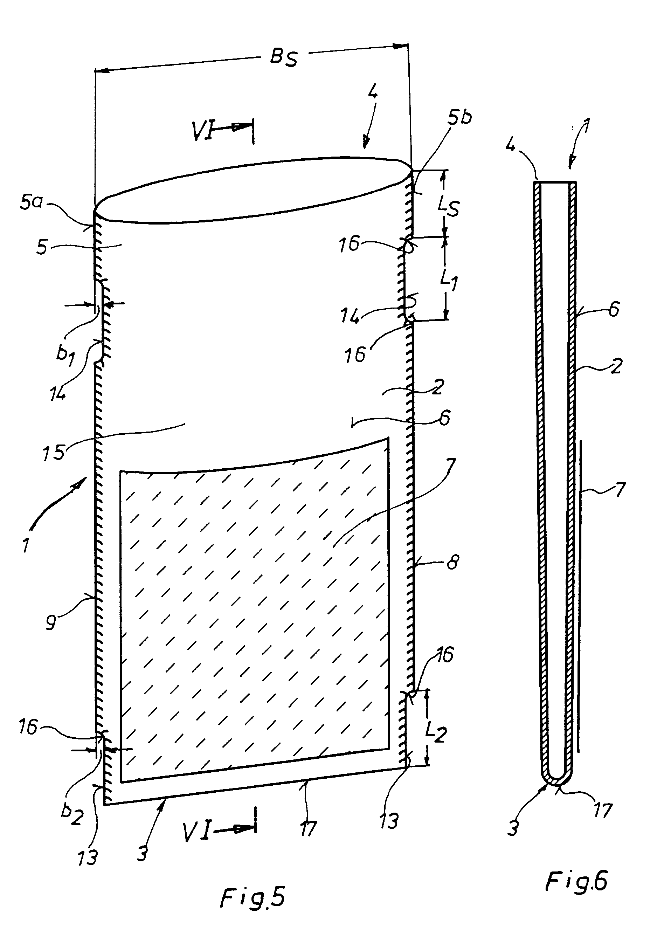

Im Ausführungsbeispiel der Figuren 5 und 6 sind die Seitenkanten 8, 9 des Operationstischbezuges 1 in der Nähe des Bodenbereiches 3 und unmittelbar unterhalb des Stulpenrandes 5 mit nach innen gerichteten Versatzbereichen 13, 14 versehen, wobei der Stulpenrand 5 die gleiche Breite B_{S} wie der zwischen den beiden Versatzbereichen 13, 14 befindliche Mittenbereich 15 des Folienschlauches 2 aufweist. Die Länge L₁ der Versatzbereiche 14 der Seitenkanten 8, 9 unterhalb des Stulpenrandes 5 ist geringfügig größer als die Länge L_{S} des Stulpenrandes 5, während die Länge L₂ der Seitenkanten 8, 9 in der Nähe des Bodenbereiches 3 entweder gleich oder geringfügig kleiner oder größer als die Länge L_{S} des Stulpenrandes 5 ist. Ferner ist die Breite b₁ der Versatzbereiche 14 unterhalb des Stulpenrandes 5 gleich der Breite b₂ der Versatzbereiche 13 in der Nähe des Bodenbereiches 3.

Wie ferner aus den Figuren 5, 7 und 9 entnommen werden kann, gehen die Enden der Versatzbereiche 13, 14 zu den angrenzenden, vorspringenden Seitenkanten 8, 9 des Mittenbereiches 15 und des Stulpenrandes 5 in Form einer Abrundung 16, z.B. eines Viertelkreises, über. Dadurch wird die Einreißgefahr infolge von Kerbspannungen sowohl beim Herstellungsverfahren als auch beim Überstreifen der Operationstischbezüge vermindert.

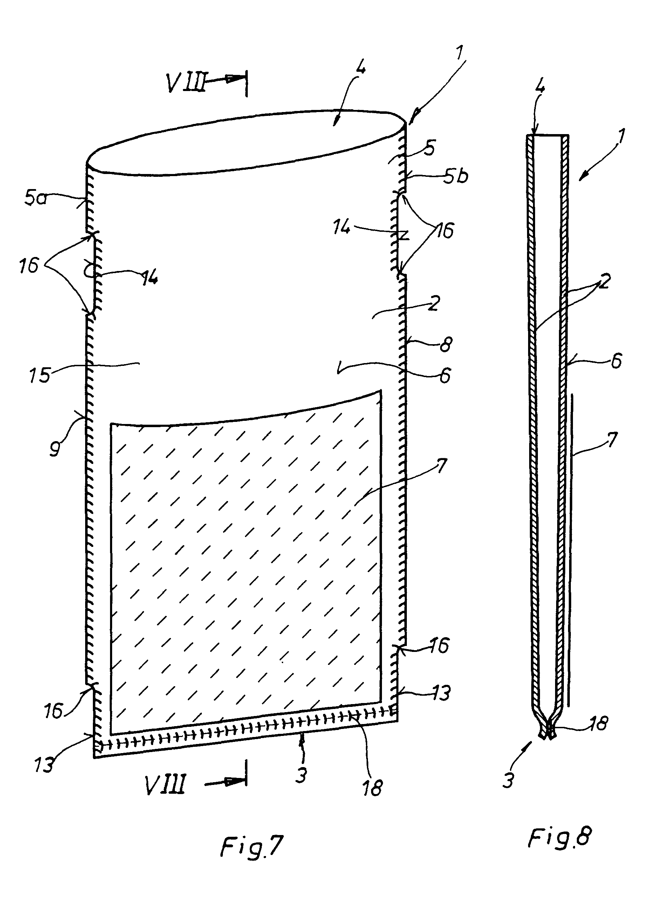

Der Unterschied zwischen der Ausführungsform gemäß den Figuren 5 und 6 und der Ausführungsform der Figuren 7 bis 10 beruht darin, daß in ersterer der Bodenbereich 3 durch eine Faltkante 17, hingegen bei letzterer durch eine Schweißnaht 18 verschlossen ist.

Bei sämtlichen Ausführungsformen der Figuren 1 bis 10 werden die Seitenkanten 8, 9 in den Versatzbereichen 13, 14 durch Trennschweißungen und am Stulpenrand 5 sowie im Mittenbereich 15 durch eine Schweißnaht mit dazwischen befindlicher Perforationschweißnaht verschlossen, auf die noch an anderer Stelle eingegangen wird.

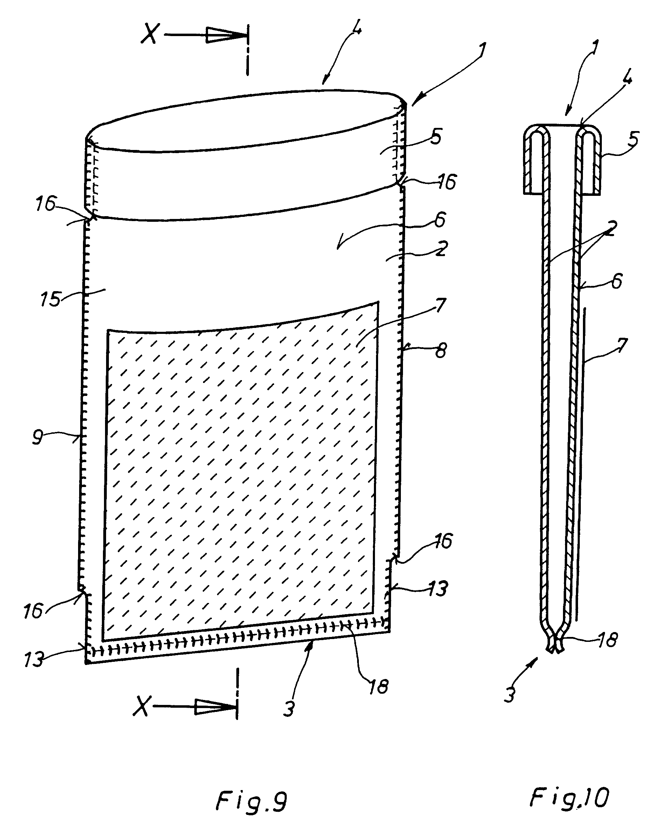

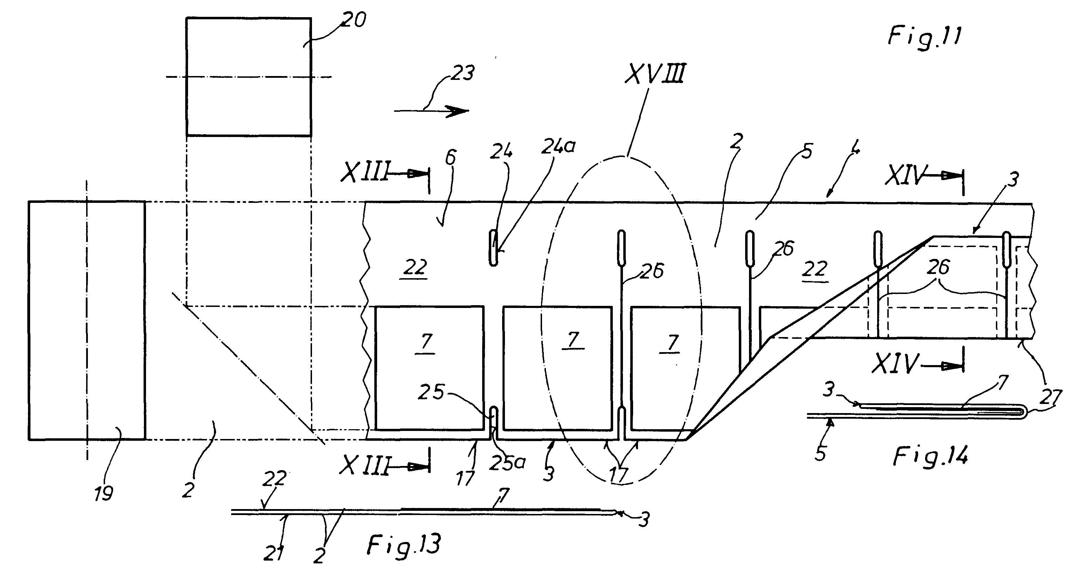

Gemäß den Figuren 9 und 10 ist der Stulpenrand 5 der Figuren 7 und 8 derart nach außen umgeschlagen, daß er mit seiner Länge L_{S} etwa die Länge L₁ der Versatzbereiche 14 übergreift. Dadurch kann beim Einfalten der Bodenbereich 3 reibungsarm zwischen die Innenseite des Stulpenrandes 5 und die Oberfläche des Folienschlauches 1 geschoben werden, um das Faservlies 7 vor infektiösen Keimen zu schützen.

In sämtlichen Figuren 1 bis 10 sowie auch in den nachfolgend zu beschreibenden Figuren 11 bis 20 sind übereinstimmende Teile mit gleichen Bezugsziffern bezeichnet.

Wie man aus den Figuren 5 bis 10 in Verbindung mit den Figuren 1 und 2 ersehen kann, sind die Versatzbereiche 13, 14 so ausgebildet, daß sie sich über die Auflagefläche 10 eines Operationstisches 11 ziehen lassen können. Während in Höhe dieser Versatzbereiche 13, 14 mit einer erhöhten Friktion beim Überziehen über die Auflagefläche 10 zu rechnen ist, wird dieser Vorgang in dem mit seinen Seitenkanten 8, 9 verbreiterten Mittenbereich 15 erheblich erleichtert. Beim Stand der Technik ist dies genau umgekehrt der Fall, wodurch auch die Handhabung des erfindungsgemäßen Operationstischbezuges 1 verbessert wird.

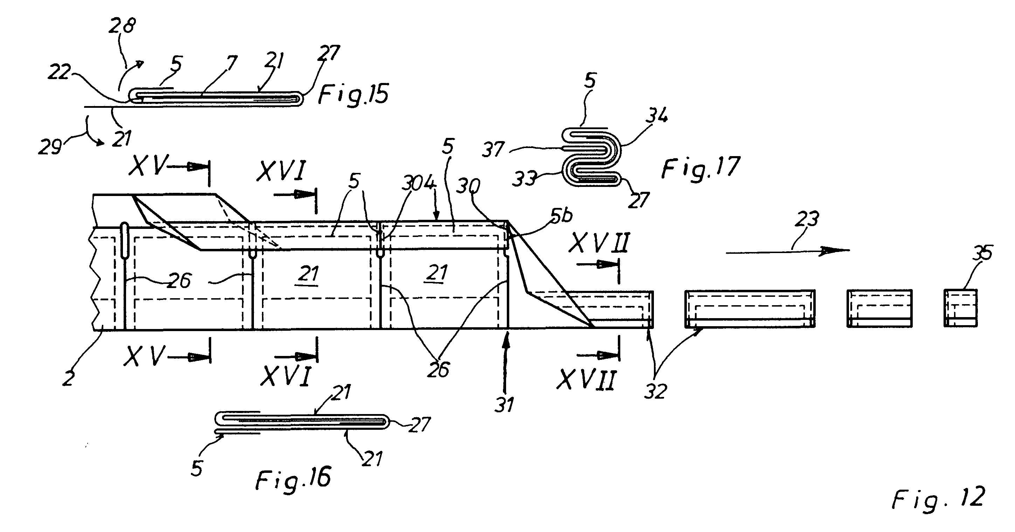

Nachfolgend wird an Hand der Figuren 11 und 12 das erfindungsgemäße Verfahren zur Herstellung eines Operationstischbezuges beschrieben, wobei mit den Figuren 1 bis 10 übereinstimmende Teile in den Figuren 11 bis 20 mit gleichen Bezugsziffern bezeichnet sind.

Von einer Vorratsrolle 19 wird der Folienschlauch 2 abgezogen, der in diesem Fall bereits aus einer einlagigen Kunststoffolie hergestellt worden ist, die exakt in ihrer Mitte parallel zur Transportrichtung gemäß dem Pfeil 23 in der Nähe des Bodenbereiches 3 der späteren Operationstischbezüge 1 längsgefalzt ist. Dadurch ist der Bodenbereich 3 bereits an dieser Seite geschlossen, hingegen am Kopfbereich 4 geöffnet.

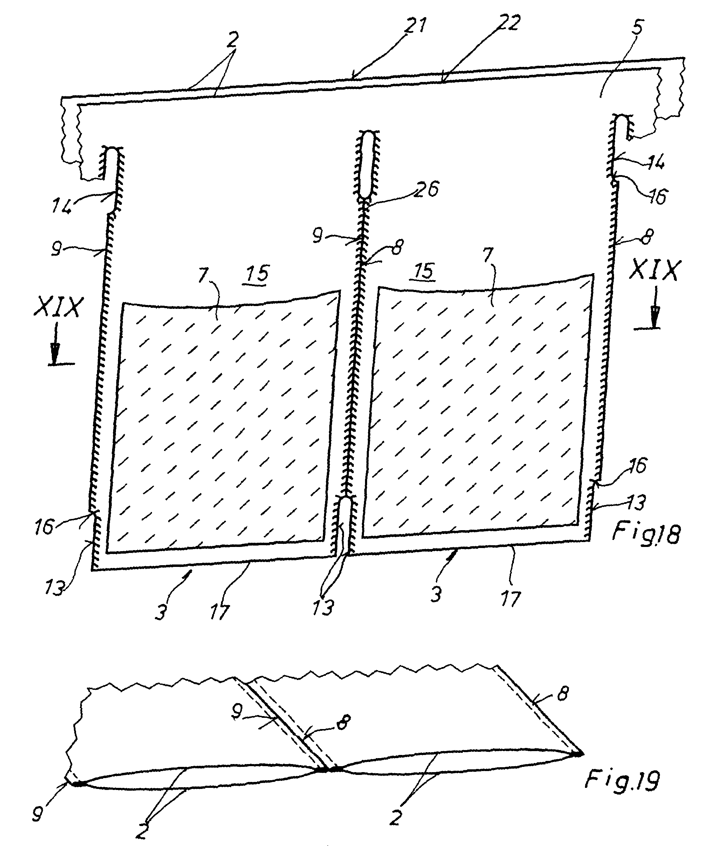

Von einer weiteren Vorratsrolle 20 läuft Faservlies zu, welches in einzelnen Zuschnitten 7 auf die Oberseite 6 des Folienschlauches 2 geklebt wird. Dieser Folienschlauch 2 wird gemäß den Figuren 18 und 19 von einer in der Zeichenebene der Fig. 11 untenliegenden Folienbahn 21 und einer darüber gefalteten zweiten Folienbahn 22 gebildet, so, wie es aus den Figuren 13 und 18 entnommen werden kann. Zur Bildung der zu den Figuren 5 bis 10 beschriebenen Versatzbereiche 13, 14 wird der Folienschlauch 2 an den späteren Seitenkanten 8, 9 des Operationstischbezuges 1 unterhalb des Stulpenrandes 5 und in der Nähe des Bodenbereiches 3 einer Trennschweißung unterzogen, wodurch die relativ kleinen, länglich ovalen Abfallbereiche 24, 25 mittels einer Trennschweißung herausgetrennt werden. Die Trennschweißung hat zur Folge, daß die beiden Folienbahnen 21, 22 gemäß Fig. 11 an den Rändern 24a, 25a der herausgetrennten Bereiche 24, 25 dicht miteinander verbunden sind.

Im darauffolgenden Fertigungsschritt, der jedoch auch mit dem vorausgegangenen gleichzeitig erfolgen könnte, wird zwischen den beiden Abfallbereichen 24, 25 eine zur Transportrichtung gemäß Pfeil 23 quer verlaufende Perforationsschweißnaht 26 angebracht und gleichzeitig zu deren beiden Seiten die beiden Folienbahnen 21, 22 in unmittelbarer Nähe dieser Perforationsschweißnaht 26 durch Schweißung miteinander verbunden und damit an diesen Stellen verschlossen.

In der rechten Bildhälfte der Fig. 11 wird der Folienschlauch 2 einer ersten Längsfalzung unterzogen, wodurch der Bodenbereich 3 unterhalb des späteren Stulpenrandes 5 in Anlage gelangt und die Zuschnitte der Faservliese 7 in eine geschützte Position eingeschlagen werden. Nach der Erstellung dieses Längsfalzes 27 bleiben die einzelnen Zuschnitte der späteren Operationstischbezüge 1 sowohl an ihren Perforationsschweißnähten 26 als auch durchgehend im Kopfbereich 4 verbunden, so, wie es aus Fig. 14 zu entnehmen ist.

Gemäß Fig. 12 in Verbindung mit den Figuren 15 und 16 wird sodann die Folienbahn 22 in Richtung des Pfeiles 28 und hiernach die Folienbahn 21 in Richtung des Pfeiles 29 zur Bildung des Stulpenrandes 5 gefalzt. Hiernach wird ein jeder Stulpenrand 5 an seinen beiden Seitenkanten 5a, 5b (s. Figuren 5 und 7) mit Perforationsschweißnähten 30 versehen und zugleich zu beiden Seiten davon durch durchgehende Schweißnähte verschlossen. Bis zur Fertigungsphase gemäß dem Pfeil 31 von Fig. 12 sind somit die einzelnen Zuschnitte der Operationstischbezüge 1 an ihren Perforationsschweißnähten 26 im Mittenbereich 15 sowie durch die weiteren Perforationsschweißnähte 30 an ihren Stulpenrändern 5 miteinander verbunden. Dadurch können beim Vorzug des Folienschlauches 2 in Transportrichtung des Pfeiles 23 keine zu Ausschußprodukten führende Verzugsspannungen auftreten, weil der Folienschlauch 2 über fast die gesamte Länge der Seitenkanten 8, 9 mit seinen übereinanderliegenden Folienbahnen 21, 22 gleichmäßig und mit kontinuierlichen Zugkräften durch die nicht dargestellten Vorzugsrollen oder Bänder weitertransportiert werden kann. Hiernach werden die einzelnen Zuschnitte der Operationstischbezüge gleichzeitig, z.B. durch Beschleunigungsrollen, sowohl in ihrem Mittenbereich 15 als auch am Stulpenrand 5 an ihren Perforationsschweißnähten 26, 30 zu Einzelprodukte 32 getrennt, die nach einer zweimaligen Längsfalzung gemäß den Falzen 33, 34 nach Fig. 17 noch mindestens einer zweimaligen Querfalzung unterzogen werden, bis sie auf die Form 35 eines anwendungsgerechten Faltpäckens 35 verkleinert worden sind.

Aus Fig. 17 ist auch das zum Schutz der darunterliegenden Bereiche des Operationstischbezuges 1 eingeschobene Metallblech 37 ersichtlich, wenn die Perforationsschweißnaht 30 am Stulpenrand 5 erst in Höhe des Pfeiles 31 erstellt werden soll.

Nach einer weiteren Alternative gestattet es das erfindungsgemäße Verfahren, die in der Fertigungsphase bei 31 noch zusammenhängenden Zuschnitte der einzelnen Operationstischbezüge 1 an ihren Perforationsschweißnähten 26, 30 verbunden zu belassen und auf eine nicht dargestellte Rolle aufzuwickeln, von der sie sodann als Abreißprodukte für die bekannte Set-Packerei entnommen werden.

Die Figur 18 zeigt den vergrößerten Ausschnitt XVIII von Fig. 11 zweier im Kopfbereich 4 noch zusammenhängender Folienbahnen 21, 22, die an den Seitenkanten 8, 9 im Mittenbereich 15 durch eine Perforationsschweißnaht 26 verbunden sind. Diese Operationstischbezüge 1 sind an ihrem Bodenbereich 3 durch die aus Fig. 5 ersichtliche Faltkante 17 verschlossen.

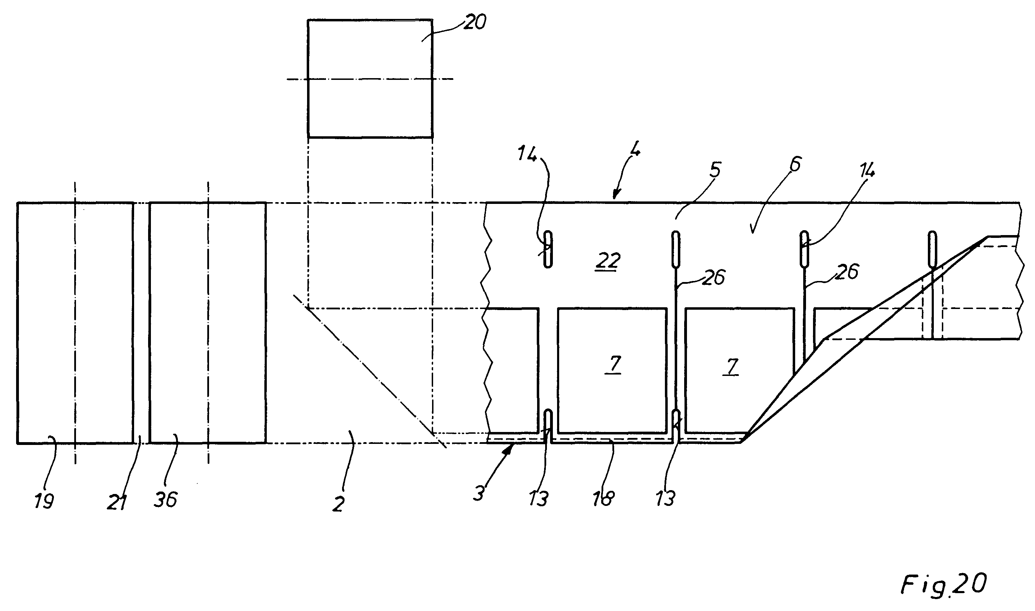

Gemäß Fig. 20 wird der Folienschlauch 2 aus zwei kongruent übereinanderliegenden sowie von zwei Vorratsrollen 19, 36 abgezogenen Einzelfolien 21, 22 gebildet, die am Bodenbereich 3 mit einer durchgehenden Schweißnaht 18 miteinander verbunden sind, so, wie es aus den Figuren 7 bis 10 hervorgeht. Die dort abgebildeten Operationstischbezüge sind daher aus Folienschläuchen 2 nach dem Verfahren gemäß Fig. 20 gebildet.

### Bezugszeichenliste:

- Operationstischbezug: 1
- Folienschlauch: 2
- Bodenbereich: 3
- Kopfbereich: 4
- Stulpenrand: 5
- Seitenkanten des Stulpenrandes 5: 5a, 5b
- Oberseite des Operationstischbezuges 1: 6
- Faservlies: 7
- Seitenkanten des Operationstischbezuges 1: 8, 9
- Auflagefläche: 10
- Kante der Auflagefläche 10: 10a
- Operationstisch: 11
- Pfeile: 12, 23, 28, 29, 31
- Versatzbereiche: 13, 14
- Mittenbereich: 15
- Abrundung: 16
- Faltkante: 17
- Schweißnaht: 18
- Vorratsrollen: 19, 20, 36
- Folienbahnen: 21, 22
- Abfallbereiche: 24, 25
- Ränder: 24a, 25a
- Perforationsschweißnähte: 26, 30
- Längsfalz: 27
- Einzelprodukt: 32
- Falze: 33, 34
- Faltpäckchen: 35
- Metallblech: 37
- Breite des Versatzbereiches 14: b₁
- Breite des Versatzbereiches 13: b2
- Länge des Versatzbereiches 14: L₁
- Länge des Versatzbereiches 13: L₂
- Länge des Stulpenrandes 5: L_{S}
- Breite des Stulpenrandes 5: B_{S}

## Patentansprüche

1. Operationstischbezug, bestehend aus einem Folienschlauch von thermoplastischem Kunststoff mit einem geschlossenen Bodenbereich und einem offenen, mit einem umlaufenden Stulpenrand versehenen Kopfbereich, dessen Seitenkanten durch Schweißnähte verschlossen sind, **dadurch gekennzeichnet**, daß die Seitenkanten (8, 9) in der Nähe des Bodenbereiches (3) und unmittelbar unterhalb des Stulpenrandes (5) mit nach innen gerichteten Versatzbereichen (13, 14) versehen sind und der Stulpenrand (5) die gleiche Breite (B_{S}) wie der zwischen den beiden Versatzbereichen (13, 14) befindliche Mittenbereich (15) des Folienschlauches (2) aufweist.

2. Operationstischbezug nach Anspruch 1, **dadurch gekennzeichnet**, daß die Länge (L₁) der Versatzbereiche (14) in den Seitenkanten (8, 9) unterhalb des Stulpenrandes (5) größer als die Länge (L_{S}) des Stulpenrandes (5) ist.

3. Operationstischbezug nach Anspruch 1 oder 2, **dadurch gekennzeichnet**, daß die Länge (L₂) der Versatzbereiche (13) in den Seitenkanten (8, 9) in der Nähe des Bodenbereiches (3) entweder gleich oder kleiner oder größer als die Länge (L_{S}) des Stulpenrandes (5) ist.

4. Operationstischbezug nach einem der Ansprüche 1 bis 3, **dadurch gekennzeichnet,** daß die Enden der Versatzbereiche (13, 14) zu den angrenzenden, vorspringenden Seitenkanten (8, 9) des Mittenbereiches (15) und des Stulpenrandes (5) in Form einer Abrundung (16), z.B. eines Viertelkreises, übergehen.

5. Operationstischbezug nach einem der Ansprüche 1 bis 4, **dadurch gekennzeichnet**, daß die Breite (b₁) der Versatzbereiche (14) unterhalb des Stulpenrandes (5) gleich der Breite (b₂) der Versatzbereiche (13) in der Nähe des Bodenbereiches (3) ist.

6. Operationstischbezug nach einem der Ansprüche 1 bis 5, **dadurch gekennzeichnet**, daß sein Bodenbereich (3) entweder eine Faltkante (17) aufweist oder durch eine Schweißnaht (18) verschlossen ist.

7. Verfahren zur Herstellung eines Operationstischbezuges aus einem Folienschlauch aus thermoplastischem Kunststoff, der an der den Bodenbereich des Operationstischbezuges bildenden Längskante geschlossen und an der den Kopfbereich bildenden Längskante geöffnet ist und der in Transportlängsrichtung durch Vorzugsrollen oder Bänder gezogen, sodann mit quer zur Transportrichtung verlaufenden Schweißnähten und Ausnehmungen versehen wird, wonach der Folienschlauch nach einer Längsfalzung im Mittenbereich sowie durch zwei zu entgegengesetzten Seiten umgeschlagenen Längsfalze an seinem Kopfbereich mit einem Stulpenrand versehen wird, dessen Seitenkanten durch je eine Schweißnaht verbunden werden**, gekennzeichnet durch** folgende Merkmale:
a) Der Folienschlauch (2) wird zur Bildung von Versatzbereichen (13, 14) an den Seitenkanten (8, 9) des Operationstischbezuges (1) unterhalb des Stulpenrandes (5) und in der Nähe des Bodenbereiches (3) einer Trennschweißung unterzogen,
b) im Mittenbereich (15) der Seitenkanten (8, 9) zwischen den beiden Versatzbereichen (13, 14) wird der Folienschlauch (2) mit einer quer zu seiner Längstransportrichtung (23) verlaufenden Perforationsschweißnaht (26) versehen und zu deren beiden Seiten durch Schweißen verschlossen,
c) der Stulpenrand (5) wird vor Trennung der einzelnen Operationstischbezüge (1) an seinen beiden Seitenkanten (5a, 5b) mit Perforationsschweißnähten (30) versehen und zu beiden Seiten davon durch Schweißen verschlossen.

8. Verfahren nach Anspruch 7, **dadurch gekennzeichnet**, daß die Perforationsschweißnähte (26, 30) im Mittenbereich (15) und am Stulpenrand (5) der Operationstischbezüge (1) gleichzeitig getrennt werden.

9. Verfahren nach den Ansprüchen 7 und 8, **dadurch gekennzeichnet**, daß die Operationstischbezüge (1) nach ihrer Trennung durch weitere Längs- und Querfalzungen (33, 34) zu einem anwendungsgerechten Faltpäckchen (35) verkleinert werden.

10. Verfahren nach Anspruch 7, **dadurch gekennzeichnet**, daß die zusammenhängenden Operationstischbezüge (1) nach Herstellung der Perforationsschweißnähte (26, 30) im Mittenbereich (15) und am Stulpenrand (5) auf eine Rolle aufgewickelt werden, von welcher sie nacheinander an ihren Perforationsschweißnähten (26, 30) im Mittenbereich (15) und am Stulpenrand (5) abreißbar sind.

11. Verfahren nach einem der Ansprüche 7 bis 10, **dadurch gekennzeichnet**, daß der Stulpenrand (5) erst nach einer weiteren Längsfalzung (33) unter Zwischenschiebung eines schützenden Metallbleches (37) zwischen dem Mittenbereich (15) und dem darauf längsgefalzten Stulpenrand (5) mit einer Perforationsschweißnaht (30) versehen und beidseitig davon durch Schweißen verschlossen wird.

12. Verfahren nach einem der Ansprüche 7 bis 11, **dadurch gekennzeichnet**, daß der Folienschlauch (2) aus einer einlagigen Kunststoffolie hergestellt wird, die exakt in ihrer Mitte (17) parallel zur Transportrichtung (23) gefalzt wird.

13. Verfahren nach einem der Ansprüche 7 bis 11, **dadurch gekennzeichnet**, daß der Folienschlauch (2) aus zwei kongruent übereinandergefügten Einzelfolien (21, 22) gebildet wird, die am Bodenbereich (3) mit einer durchgehenden Schweißnaht (18) parallel zur Transportlängsrichtung (23) versehen werden.

## Claims

1. A cover for an operating table, comprising a foil tube made of thermoplastic plastic with a closed foot region and an open head region comprising a surrounding folded-back margin, with the lateral edges of said cover for an operating table being closed off by weld seams, characterised in that the lateral edges (8, 9) near the foot region (3) and directly below the folded-back margin (5) comprise offset regions (13, 14) directed inward, and that the width (B_{S}) of the folded-back margin (5) is the same as the width of the middle region (15) of the foil tube (2) said middle region (15) being situated between the two offset regions (13, 14).

2. The cover for an operating table according to claim 1, characterised in that the length (L₁) of the offset regions (14) in the lateral edges (8, 9) below the folded-back margin (5) exceeds the length (L_{S}) of the folded-back margin (5).

3. The cover for an operating table according to claim 1 or 2, characterised in that the length (L₂) of the offset regions (13) in the lateral edges (8, 9) near the foot region (3) are either the same or smaller or larger than the length (L_{S}) of the folded-back margin (5).

4. The cover for an operating table according to one of claims 1 to 3, characterised in that the transition from the ends of the offset regions (13, 14) to the adjoining protruding lateral edges (8, 9) of the middle region (15) and the folded-back margin (5) is in the form of a rounding (16), e.g. of a quarter circle.

5. The cover for an operating table according to one of claims 1 to 4, characterised in that the width (b₁) of the offset regions (14) below the folded-back margin (5) is equal to the width (b₂) of the offset regions (13) near the foot region (3).

6. The cover for an operating table according to one of claims 1 to 5, characterised in that its foot region (3) comprises either a folding edge (17) or is closed off by a weld seam (18).

7. A method of manufacturing a cover for an operating table from a foil tube made of thermoplastic plastic, said cover being closed at the longitudinal edge forming the foot region of the cover for an operating table, and open at the longitudinal edge forming the head region; said cover in longitudinal direction of transport being pulled through tension rollers or belts before weld seams extending across the direction of transport, and recesses are made, whereupon the foil tube after longitudinal folding in the middle region as well as by two longitudinal folds in opposite directions, being applied at its head region, are given a folded-back margin whose lateral edges are connected by one weld seam each, characterised by the following features:
a) to form offset regions (13, 14) at the lateral edges (8, 9) of the cover (1) for an operating table below the folded-back margin (5) and near the foot region (3), the foil tube (2) is subjected to separation welding;
b) in the middle region (15) of the lateral edges 8, 9) between the two offset regions (13, 14) the foil tube (2) is given a perforation separation weld seam (26) extending across the direction of longitudinal transport (23), with the two sides of said foil tube being closed off by welding;
c) prior to separation of individual covers (1) for an operating table, perforation weld seams (30) are applied to the two lateral edges (5a, 5b) of the folded-back margin (5), at both sides closing them off by welding.

8. The method according to claim 7, characterised in that the perforation weld seams (26, 30) in the middle region (15) and at the folded-back margin (5) of the covers (1) for an operating table are separated at the same time.

9. The method according to claims 7 and 8, characterised in that after their separation, each cover (1) for an operating table is reduced in size by further longitudinal and transverse folds (33, 34) to become a folded parcel (35) ready for use.

10. The method according to claim 7, characterised in that after application of the perforation weld seams (26, 30) in the middle region (15) and at the folded-back margin (5) the joined covers (1) for an operating table are rolled onto a roll from which said covers can be torn off one by one, at their perforation weld seams (26, 30) in the middle region (15) and at the folded-back margin (5).

11. The method according to one of claims 7 to 10, characterised in that the folded-back margin (5) receives a perforation weld seam (30) only after further longitudinal folding (33) where a protective metal sheet (37) is placed between the middle region (15) and the folded-back margin (5) which is longitudinally folded on said metal sheet (37), and is closed by welding, on both sides of said perforation weld seam (30).

12. The method according to one of claims 7 to 11, characterised in that the foil tube (2) is made from a single-layer plastic foil which is folded exactly in the middle (17), parallel to the direction of transport (23).

13. The method according to one of claims 7 to 11, characterised in that the foil tube (2) is made from two individual foils (21, 22) congruently placed one on top of the other, said individual foils (21, 22) receiving a continuous weld seam (18) in the foot region (3), parallel to the longitudinal direction of transport (23).

## Revendications

1. Couverture pour table d'opération comprenant un tube soufflé en matière thermoplastique avec une partie de fond fermée et une partie de tête ouverte et pourvue sur son pourtour d'un bord embouti, dont les bords latéraux sont fermés au moyen de joints soudés, caractérisée en ce que les bords latéraux (8, 9) sont pourvus à la hauteur de la partie de fond (3) et directement en dessous du bord embouti (5) de parties en déport (13, 14) et en ce que le bord embouti (5) est de même largeur (B_{S}) que la partie médiane (15) du tube soufflé (2) située entre les deux parties en déport (13, 14).

2. Couverture pour table d'opération selon la revendication 1 caractérisée en ce que sur les bords latéraux (8, 9) en dessous du bord embouti (5), la longueur (L₁) des parties en déport (14) est supérieure à la longueur (L_{S}) de celui-ci (5).

3. Couverture pour table d'opération selon la revendication 1 ou 2 caractérisée en ce que sur les bords latéraux (8, 9) à proximité de la partie de fond (3), la longueur (L₂) des parties en déport (13) est égale ou inférieure ou supérieure à la longueur (L_{S}) du bord embouti (5).

4. Couverture pour table d'opération selon l'une des revendications 1 à 3 caractérisée en ce que les extrémités des parties en déport (13, 14) se prolongent vers les bords latéraux (8, 9) adjacents et saillants de la partie médiane (15) et du bord embouti (5) en formant un arrondi (16), un quart de cercle par exemple.

5. Couverture pour table d'opération selon l'une des revendications 1 à 4 caractérisée en ce que la largeur (b₁) des parties en déport (14) en dessous du bord embouti (5) est égale à la largeur (b₂) des parties en déport (13) à proximité de la partie de fond (3).

6. Couverture pour table d'opération selon l'une des revendications 1 à 5 caractérisée en ce que sa partie de fond (3) présente un bord replié (17) ou est fermée au moyen d'un joint soudé (18).

7. Procédé de fabrication d'une couverture pour table d'opération à partir d'un tube soufflé en matière thermoplastique, lequel est fermé à hauteur de son bord longitudinal formant la partie de fond de la couverture pour table d'opération et ouvert à hauteur de son bord longitudinal formant la partie de tête et lequel, tiré dans l'axe longitudinal de déplacement au moyen de rouleaux d'avancement ou de bandes, est ainsi pourvu de joints soudés et d'évidements transversaux à l'axe de déplacement, selon lequel le tube soufflé est pourvu, suite à un pliage longitudinal dans la partie médiane et au moyen de deux plis longitudinaux situés dans la partie de tête et repliés pour former deux côtés opposés, d'un bord embouti dont les bords latéraux sont respectivement assemblés au moyen d'un joint soudé, et caractérisé comme suit :
a) Le tube soufflé (2) subit une séparation-soudage de sorte à former des parties en déport (13, 14) sur les bords latéraux (8, 9) de la couverture pour table d'opération (1) en dessous du bord embouti (5) et à proximité de la partie de fond (3),
b) dans la partie médiane (15) des bords latéraux (8, 9) entre les parties en déport (13, 14), le tube soufflé (2) est pourvu d'une soudure perforée (26) transversale à son axe longitudinal de déplacement (23) et obturé par soudure des deux côtés de celle-ci,
c) en amont de la séparation par unité des couvertures pour table d'opération (1), le bord embouti (5) est sur ses deux bords latéraux (5a, 5b) pourvu de soudures perforées (30) et obturé par soudure des deux côtés de celles-ci.

8. Procédé selon la revendication 7 caractérisé en ce que les soudures perforées (26, 30) situées dans la partie médiane (15) et sur le bord embouti (5) de la couverture pour table d'opération (1) sont séparées simultanément.

9. Procédé selon les revendications 7 et 8 caractérisé en ce qu'une fois séparées, les couvertures pour table d'opération (1) sont réduites à un petit paquet plié (35) facile à utiliser, au moyen d'autres opérations de pliage longitudinal et transversal (33, 34).

10. Procédé selon la revendication 7 caractérisé en ce qu'après la réalisation des soudures perforées (26, 30) dans la partie médiane (15) et sur le bord embouti (5), les couvertures pour table d'opération (1) non séparées sont enroulées sur un rouleau d'où elles sont détachables à l'unité le long de leurs soudures perforées (26, 30) sur la partie médiane (15) et le bord embouti (5).

11. Procédé selon l'une des revendications 7 à 10 caractérisé en ce que le bord embouti (5) n'est pourvu d'une soudure perforée (30) et obturé par soudure des deux côtés de celle-ci qu'après un nouveau pliage avec intercalage d'une tôle métallique (37) de protection entre la partie médiane (15) et le bord embouti (5) replié sur cette dernière dans un sens longitudinal (33).

12. Procédé selon l'une des revendications 7 à 11 caractérisé en ce que le tube soufflé (2) est réalisé à partir d'une feuille plastique unicouche qui est pliée exactement dans son milieu (17) parallèlement à l'axe de déplacement.

13. Procédé selon l'une des revendications 7 à 11 caractérisé en ce que le tube soufflé (2) est formé par deux feuilles individuelles (21, 22) superposées de façon congruente, lesquelles sont pourvues à la hauteur de la partie de fond (3) d'un joint soudé (18) continu parallèle à l'axe de déplacement (23).
